# EUROPEAN PATENT APPLICATION

(11) **EP 3 492 324 A1**
(43) Date of publication of application: **05.06.2019**
(21) Application number: 17834064.2
(22) Date of filing: 14.07.2017
(51) Int. Cl.: B60R 21/26

(54) **AIR BAG CONTROL DEVICE AND AIR BAG CONTROL METHOD**

(30) Priority: 28.07.2016 JP 2016148591
(71) Applicant: Joyson Safety Systems Japan K.K., Shinagawa-ku Tokyo 1400002 (JP)
(72) Inventor: NISHIMURA, Jun, Tokyo 1400002 (JP); AZUMA, Junji, Tokyo 1400002 (JP)
(74) Representative: Banzer, Hans-Jörg
(86) International application number: PCT/JP2017/025663
(87) International publication number: WO 2018/021056

(57) **Abstract**

The purpose of the present invention is to provide an air bag control device and method which are able to address changes due to aging in accordance with environmental conditions in which an air bag device is installed. An air bag control device and method for activating an inflator of an air bag on the basis of an emergency detection signal, said air bag control device and method being characterized in that environmental load of the air bag device is integrated, and when the integrated value of the environmental load has reached a predetermined value, either an alert is issued and/or the inflator is not activated even when a vehicular emergency is detected. The environmental load includes temperature and humidity, for example.

## Description

### Technical Field

The present invention relates to an airbag control device and a method of controlling activation of an airbag device installed in a moving object such as a vehicle.

### Background Art

An airbag device for a vehicle and the like is configured to activate an inflator to restrain an occupant when an emergency such as collision or rollover of the vehicle is detected.

Patent Document 1 describes a device used for disabling an airbag ignition device which disables the ignition device of an airbag device according to whether a child seat is mounted.

Patent Document 1: JP-T-2003-519044

### Summary of Invention

It is presently assumed that a main cause of changes in inflator output characteristics over time is changes in properties of explosive. The changes in the properties of the explosive are caused by temperature changes and humidity around an airbag, and elapse of time. There is a limit to semi-permanently prevent output changes due to the changes in the properties of the explosive, so that the output characteristics may increase or decrease over time.

An object of the present invention is to provide an airbag control device and an airbag control method which can cope with changes over time in accordance with environmental conditions in which an airbag device is installed.

An airbag control device according to the present invention includes an emergency detection unit, an inflator activation unit configured to activate an inflator of an airbag device based on an emergency detection signal of the emergency detection unit, an environmental load detection unit configured to detect an environmental load of the airbag device, an integration unit configured to integrate the environmental load detected by the detection unit, and a countermeasure activation unit configured to perform at least one of issuing an alert and disabling the inflator activation unit when an environmental load integrated value of the integration unit reaches a predetermined value.

According to an aspect of the present invention, the environmental load includes at least one of temperature, humidity, vehicle traveling distance, vehicle vibration, time elapse from vehicle manufacturing, and a vehicle presence area.

According to an aspect of the present invention, the environmental load includes temperature and humidity.

According to an aspect of the present invention, the detection unit of at least one of temperature and humidity includes a sensor provided in a vehicle or a reception unit configured to receive temperature data or humidity data transmitted from a communication unit.

According to an aspect of the present invention, the airbag control device further comprises a load index storage unit configured to store a load index set corresponding to temperature and humidity. The integration unit is configured to read the load index from the load index storage unit based on measured values of temperature and humidity at predetermined time intervals and integrate the load index to obtain the environmental load integrated value.

According to an aspect of the present invention, the countermeasure activation unit includes a communication unit configured to communicate the alert.

According to an aspect of the present invention, the countermeasure activation unit is configured to issue an alert when the environmental load integrated value reaches a first predetermined value, and disable the inflator activation unit or disable the inflator activation unit and issue an alert when the environmental load integrated value reaches a second predetermined value.

According to an aspect of the present invention, the countermeasure activation unit is configured to issue an alert and disable the inflator activation unit when the environmental load integrated value reaches a predetermined value.

According to an aspect of the present invention, the alert includes an alert prompting replacement of the inflator and an alert indicating that the airbag device is disabled.

A method of forming the airbag control device of the present invention includes assembling the environmental load detection unit, the integration unit, and the countermeasure activation unit into an existing emergency detection unit and an existing inflator activation unit.

An airbag control method according to the present invention is for activating an inflator of an airbag device based on an emergency detection signal and includes integrating an environmental load of the airbag device, and when an environmental load integrated value reaches a predetermined value, performing at least one of issuing an alert disabling the inflator even if a vehicle emergency is detected.

### Effect of the Invention

According to the present invention, when the airbag device mounted in the vehicle and the like is subjected to severe environmental load conditions, at least one of issuing an alert and disabling the inflator is performed at an early stage. When subjected to mild environmental load conditions, the airbag device is enabled over a long period of time.

### Brief Description of Drawings

Fig. 1 is a block diagram of an airbag control device according to an embodiment.
Fig. 2 is an environmental load data structure diagram of the airbag control device according to an embodiment.
Fig. 3 is a flowchart illustrating operation of the airbag control device.

### Description of Embodiments

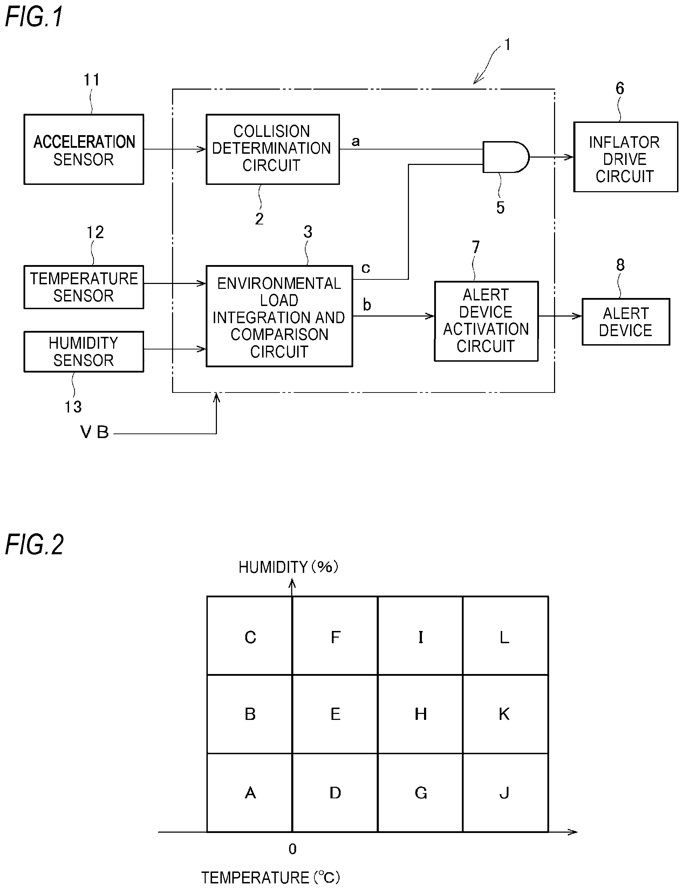

Hereafter, an embodiment is described with reference to the drawings. Fig. 1 is a block diagram of a vehicle airbag control device 1 according to an embodiment. The airbag control device 1 is configured to control an inflator drive circuit 6 by a collision determination circuit 2, an environmental load integration and comparison circuit 3, and an AND circuit 5, and to control an alert device 8 by an alert device activation circuit 7.

Acceleration detected by an acceleration sensor 11 is input to the collision determination circuit 2, so that a collision is determined by waveform processing of the acceleration and determination processing. An output a of the collision determination circuit is a = 0 until a collision is determined, and a = 1 when a collision is determined. The output a is input to the AND circuit 5.

Temperature detected by a temperature sensor 12 and humidity detected by a humidity sensor 13 are input to the environmental load integration and comparison circuit 3. The temperature sensor 12 and the humidity sensor 13 are installed in the vicinity of an airbag device.

A memory of the environmental load integration and comparison circuit stores a table including environmental load index data shown in Fig. 2. In the table, the temperature is divided into a plurality of stages from a low temperature side to a high temperature side, and the humidity (in this case, relative humidity) is divided into a plurality of stages from low to high. A plurality of sections A to L are set corresponding to the temperature and the humidity. An index is set for each of the sections.

In this embodiment, the temperature is divided into four stages, i.e. lower than 0°C (sections A, B, C), 0°C or higher and lower than 40°C (sections D, E, F), 40°C or higher and lower than 65°C (sections G, H, I), and 65°C or higher (sections J, K, L). The humidity is divided into three stages, i.e. lower than 30RH% (sections A, D, G, J), 30RH% or higher and lower than 60RH% (sections B, E, H, K), and 60RH% or higher (sections C, F, I, L).

In general, the higher the temperature is and the higher the humidity is, the shorter the life of the airbag device (especially an explosive of an inflator) is. However, an effect of the humidity is small when the temperature is below a freezing point. Therefore, in this embodiment, values (indices) of A to L are set as shown in Table 1. However, this setting of the values is an example and does not limit the present invention.

**[Table 1]**

| | Lower than 0°C | 0 to 40°C | 40 to 65°C | 65°C or Higher |
|---|---|---|---|---|
| 60 to 100RH% | C=0.1 | F=1.7 | 1=7.0 | L=50 |
| 30 to 60RH% | B=0.1 | E=1 | H=4.2 | K=30 |
| 0 to 30RH% | A=0 | D=0.5 | G=2.1 | J=15 |

The temperature and the humidity are read every predetermined time, and an environmental load index at that time is determined from Table 1. The environmental load index is obtained and integrated every predetermined time. Outputs of the environmental load integration and comparison circuit 3 are set to b = 0, c = 1 until an integrated value reaches a predetermined value S₀ set in advance. When the integrated value reaches the predetermined value S₀, the output is b = 1 and c is maintained as c = 1. When b = 0 changes to b = 1, the alert device activation circuit 7 activates the alert device 8 to display an alert (for example, an alert prompting replacement of the inflator). In a case of c = 1, the AND circuit 5 activates the inflator drive circuit 6 when a collision detection signal a = 1 is input from the collision determination circuit to the AND circuit 5.

When the integrated value of the environmental load index further increases to another predetermined value S₁, an output of the environmental load integration and comparison circuit 3 is set to c = 0, and b is maintained as b = 1. The output c is input to the AND circuit 5. Accordingly, when the output is c = 0, the AND circuit 5 does not activate the inflator drive circuit 6 even if the collision detection signal a = 1 is input. When the output is c = 0, it is preferable to display on the alert device 8 an alert indicating that the airbag device is disabled and an alert prompting the replacement of the inflator.

Examples of the alert device 8 include a display screen of liquid crystal and the like provided on an instrument panel, and an alert lamp. An alert sound unit which generates an alert sound or alert voice may be used together with or in place of such visual display. An alert may be transmitted to a portable communication terminal of a vehicle owner via a communication line. The inflator drive circuit of a vehicle may be disabled via the communication line.

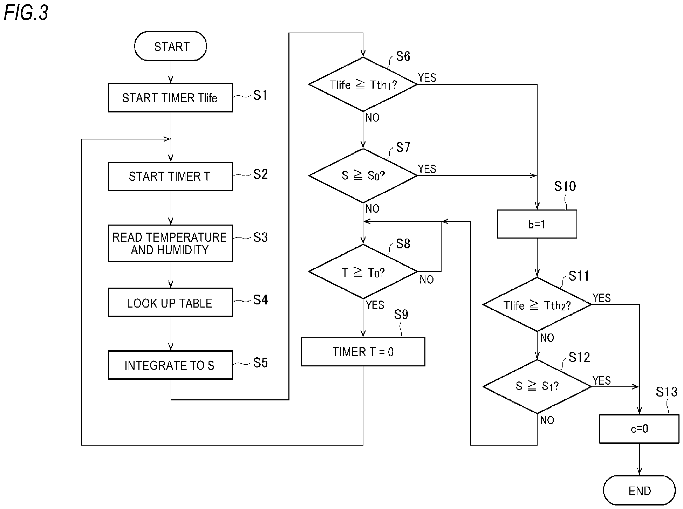

Fig. 3 is a flowchart illustrating the control described above.

A program starts when the airbag control device 1 is connected to a vehicle battery and a power supply voltage VB is applied. First, a vehicle life timer T_{life} is started (Step 1 (denoted as S1 in Fig. 3, the same also applies below)), and a timer T is started (Step 2). Then, detection temperature and detection humidity from the temperature sensor 12 and the humidity sensor 13 are read, or data of the detection temperature and the detection humidity are received from another system via a vehicle communication network (Step 3). The table in Fig. 2 (Table 1) is looked up (Step 4). Environmental load index values corresponding to the detection temperature and the detection humidity are determined from A to L, and added to an integrated value S in the memory (Step 5). Next, it is determined whether an integrated value of the vehicle life timer T_{life} is equal to or higher than a predetermined value Tₜₕ₁ (Step 6). When the integrated value of the vehicle life timer T_{life} is lower than the predetermined value Tₜₕ₁, a process proceeds to Step 7, in which it is determined whether the integrated value S in the memory is equal to or higher than a predetermined value S₀ (Step 7). When the integrated value S in the memory is lower than the predetermined value S₀, the process proceeds to Step 8 and waits until a predetermined time T₀ elapses. When the predetermined time T₀ elapses, the timer T is reset (Step 9), and the process returns to Step 2.

The alert device 8 is not activated during circulation from Steps 2 to 9. When the collision determination circuit 2 determines a collision during this period, the inflator is activated so that airbag is inflated and expanded.

When T_{life} ≥ Tₜₕ₁ is determined in Step 6, or when S ≥ S₀ is determined in Step 7, the process proceeds to Step 10 to output b = 1. As a result, the alert device 8 is activated, for example, an alert prompting the replacement of the inflator is displayed. Then, the process proceeds to Step 11.

When the vehicle lift timer T_{life} is lower than a predetermined value Tₜₕ₂ and the integrated value S is lower than the predetermined value S₁ even after b = 1 is output in Step 10 and the alert is displayed on the alert device 8, the vehicle life timer T_{life} continues integration and circulates from Steps 2 to 7, 10 to 12 and 8 to 9. The inflator drive circuit 6 is in an enabled state during this circulation period. The predetermined value Tₜₕ₂ is higher than the predetermined value Tₜₕ₁, and the predetermined value S₁ is higher than the predetermined value S₀.

When T_{life} ≥ Tₜₕ₂ is determined in Step 11, or when S ≥ S₁ is determined in Step 12, the process proceeds to Step 13 to output c = 0. As a result, the inflator drive circuit 6 is disabled. In conjunction therewith, the alert device 8 is activated to notify content prompting the replacement of the inflator and content that the airbag is disabled.

In the above description, the countermeasure activation unit causes the alert device 8 to display an alert when the vehicle life timer integrated value is T_{life} ≥ Tₜₕ₁ or the environmental load integrated value is S ≥ S₀. Thereafter, when the vehicle life timer integrated value is T_{life} ≥ Tₜₕ₂ or the environmental load integrated value is S ≥ S₁, the alert is displayed on the alert device 8 and the inflator drive circuit 6 is disabled. However, when the vehicle life timer integrated value is T_{life} ≥ Tₜₕ₂ or the environmental load integrated value is S ≥ S₁, only one of the following steps may be performed: the alert is displayed on the alert device 8 or the inflator drive circuit 6 is disabled.

The threshold value So and the threshold value S₁ may be set to the same value, or the threshold value Tₜₕ₁ and the threshold value Tₜₕ₂ may be set to the same value, so that the inflator drive circuit 6 may be disabled simultaneously with the alert display by the alert device 8.

As described above, in the embodiment, the alert from the alert device 8, when the inflator drive circuit 6 is in the enabled state, issues notification prompting of the replacement of the inflator. The alert of the alert device 8 issued when the inflator drive circuit 6 is disabled notifies the content prompting the replacement of the inflator and the content that the airbag is disabled. However, the present invention is not limited thereto, and the alert of the alert device 8 may further include other contents.

In Table 1, a maximum temperature section is set to 65°C or higher. However, a very high index may be set for a very high temperature, for example 200°C or higher. It may be determined as S ≥ S₀ when 200°C is measured for even a small number of times (for example, once or several times).

In above description, the temperature sensor 12 and the humidity sensor 13 are used. However, temperature data and humidity data transmitted by a communication unit such as the Internet may be received, and the temperature data and the humidity data may be input to the environmental load integration and comparison circuit 3. Only one of the temperature data and the humidity data may be received from the communication unit and the other may be detected by a sensor.

Detection results of the temperature sensor 12 and the humidity sensor 13 may be transmitted to a management center by using a wireless communication device capable of wireless communication. The management center monitors the environmental load integrated value of the vehicle from the received detection results, and transmits alert information to the vehicle when the integrated value exceeds a predetermined threshold value. When the communication device of the vehicle receives the alert information from the management center, the alert is displayed on the alert device 8 or the inflator drive circuit 6 is disabled. The alert information may be transmitted from the management center to the portable terminal of the vehicle owner. In such a configuration, the environmental load integration and comparison circuit 3 can be omitted from the vehicle.

In above description, the temperature and the humidity is exemplified as an environmental load. However, the environmental load is not limited thereto and may be at least one of temperature, humidity, vehicle traveling distance, vehicle vibration, elapse time from vehicle manufacturing, a vehicle presence area, and the like. However, it is preferable that the environmental load is two items of the temperature and the humidity, or is these two items and one or more of the other items.

The vehicle vibration can be detected by measuring the acceleration in a direction orthogonal to a traveling direction of the vehicle, for example, the acceleration in a vertical direction. The vehicle presence area can be detected by using GPS information.

In the present invention, the airbag control device may be configured by adding the environmental load integration and comparison circuit 3, the alert device activation circuit 7, and the like to an existing airbag control device including the acceleration sensor 11, the collision determination circuit 2, and the inflator drive circuit 6.

In the above description, collision of the vehicle is detected to activate the inflator. However, the present invention also includes a case where a rollover of the vehicle is detected to activate the inflator.

The airbag device includes a side airbag device, a curtain airbag device, a knee airbag device, and the like in addition to a driver seat airbag device, a passenger seat airbag device, and a rear seat airbag device. The airbag device also includes an airbag device other than the vehicle airbag device.

The above embodiment is an example of the present invention, and the present invention is not limited thereto.

This application is based on Japanese Patent Application 2016-148591, filed on July 28, 2016, entirety of which is incorporated herein by reference.

### Description of Reference Numerals

1 airbag control device
2 collision determination circuit
3 environmental load integration and comparison circuit

## Claims

1. An airbag control device comprising:
an emergency detection unit;
an inflator activation unit configured to activate an inflator of an airbag device based on an emergency detection signal of the emergency detection unit;
an environmental load detection unit configured to detect an environmental load of the airbag device;
an integration unit configured to integrate the environmental load detected by the detection unit; and
a countermeasure activation unit configured to perform at least one of issuing an alert and disabling the inflator activation unit when an environmental load integrated value of the integration unit reaches a predetermined value.

2. The airbag control device according to claim 1,
wherein the environmental load includes at least one of temperature, humidity, vehicle traveling distance, vehicle vibration, elapse time from vehicle manufacturing, and a vehicle presence area.

3. The airbag control device according to claim 1,
wherein the environmental load includes temperature and humidity.

4. The airbag control device according to claim 2,
wherein the detection unit of at least one of temperature and humidity includes a sensor provided in a vehicle or a reception unit configured to receive temperature data or humidity data transmitted from a communication unit.

5. The airbag control device according to claim 3 or 4, further comprising:
a load index storage unit configured to store a load index set corresponding to temperature and humidity,
wherein the integration unit is configured to read the load index from the load index storage unit based on measured values of temperature and humidity at predetermined time intervals and integrate the load index to obtain the environmental load integrated value.

6. The airbag control device according to any one of claims 1 to 5,
wherein the countermeasure activation unit includes a communication unit configured to communicate the alert.

7. The airbag control device according to any one of claims 1 to 6,
wherein the countermeasure activation unit is configured to:
issue an alert when the environmental load integrated value reaches a first predetermined value; and
disable the inflator activation unit or disable the inflator activation unit and issue an alert when the environmental load integrated value reaches a second predetermined value.

8. The airbag control device according to claim 7,
wherein the alert issued when the environmental load integrated value reaches the second predetermined value includes an alert prompting replacement of the inflator and an alert indicating that the airbag device is disabled.

9. The airbag control device according to any one of claims 1 to 6,
wherein the countermeasure activation unit is configured to issue the alert and disable the inflator activation unit when the environmental load integrated value reaches a predetermined value.

10. The airbag control device according to claim 9,
wherein the alert includes an alert prompting replacement of the inflator and an alert indicating that the airbag device is disabled.

11. A method of forming the airbag control device according to any one of claims 1 to 10,
wherein the environmental load detection unit, the integration unit, and the countermeasure activation unit are assembled into an existing emergency detection unit and an existing inflator activation unit.

12. An airbag control method of activating an inflator of an airbag device based on an emergency detection signal, the method comprising:
integrating an environmental load of the airbag device; and
when an environmental load integrated value reaches a predetermined value, performing at least one of issuing an alert and disabling the inflator even if a vehicle emergency is detected.

13. An airbag control device comprising:
an emergency detection unit;
an inflator activation unit configured to activate an inflator of an airbag device based on an emergency detection signal of the emergency detection unit;
an environmental load detection unit configured to detect an environmental load of the airbag device;
a communication unit configured to transmit the environmental load detected by the environmental load detection unit to a management center and to receive alert information from the management center when an environmental load integrated value reaches a predetermined value; and
a countermeasure activation unit configured to perform at least one of issuing an alert and disabling the inflator activation unit upon receiving the alert information.
